(19) Europäisches Patentamt
European Patent Office
Office européen des brevets

(11) Veröffentlichungsnummer: **0 275 955**
**B1**

(12) # EUROPÄISCHE PATENTSCHRIFT

(45) Veröffentlichungstag der Patentschrift: **04.07.90**

(51) Int. Cl.[5]: **C07D 249/08,** A01N 43/653

(21) Anmeldenummer: **88100610.0**

(22) Anmeldetag: **18.01.88**

(54) **Mikrobizides Mittel.**

(30) Priorität: **21.01.87 CH 214/87**

(43) Veröffentlichungstag der Anmeldung:
**27.07.88 Patentblatt 88/30**

(45) Bekanntmachung des Hinweises auf die Patenterteilung:
**04.07.90 Patentblatt 90/27**

(84) Benannte Vertragsstaaten:
**AT BE CH DE ES FR GB GR IT LI LU NL SE**

(56) Entgegenhaltungen:
**EP-A- 0 000 017**
**EP-A- 0 000 018**
**EP-A- 0 077 479**
**EP-A- 0 126 430**

(73) Patentinhaber: **CIBA-GEIGY AG, Klybeckstrasse 141, CH-4002 Basel(CH)**

(72) Erfinder: **Riebli, Peter, Bünten 17, D-4446 Buckten(CH)**
Erfinder: **Hubele, Adolf, Dr., Obere Egg 9, D-4312 Magden(CH)**

(74) Vertreter: **Zumstein, Fritz, Dr. et al, Bräuhausstrasse 4, D-8000 München 2(DE)**

**EP 0 275 955 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Mikrobizides Mittel

Die vorliegende Erfindung betrifft neue 1-Phenoxyphenyl-1-triazolylmethyl-carbinole der Formel I und ein mikrobizides Mittel, das als Wirkstoff mindestens eine Verbindung der nachstehenden Formel I oder eine Säureadditionsverbindung davon enthält, die Herstellung dieser Verbindungen oder der sie enthaltenden Mittel sowie Verfahren zur Bekämpfung oder Verhütung des Befalls von Pflanzen durch phytopathogene Mikroorganismen.

Die 1-Phenoxyphenyl-1-triazolylmethyl-carbinole entsprechen der Formel I

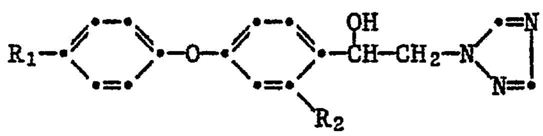

(I)

worin

$R_1$ Fluor, Chlor oder Brom und

$R_2$ Fluor, Chlor, Brom oder Methyl

bedeutet, sowie Säureadditionssalze dieser Verbindungen mit pflanzenphysiologisch verträglichen organischen und anorganischen Säuren.

Als Salzbildner mit Verbindungen der Formel I kommen alle organischen und anorganischen Säuren in Frage, soweit sie pflanzenphysiologisch verträgliche Salze bilden.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, Phosphorige Säure, Salpetersäure und organische Säuren wie Essigsäure, Trifluoressigsäure, Trichloressigsäure, Propionsäure, Weinsäure, Glykolsäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure. Diese Säuren werden nach an sich bekannten Methoden an die betreffenden freien Verbindungen der Formel I addiert.

Die Verbindungen der Formel I sind bei Raumtemperatur stabil. Sie lassen sich auf dem Agrarsektor oder verwandten Gebieten insbesondere präventiv und kurativ zur Bekämpfung von pflanzenschädigenden Mikroorganismen einsetzen. Die Wirkstoff der Formel I zeichnen sich in weiten Anwendungskonzentrationen durch eine sehr gute fungizide Wirkung und problemlose Anwendung aus.

Die Verbindungen der Formel I besitzen ausgeprägte mikrobizide Wirksamkeit. Von der Formel werden folgende 12 Verbindungen umfasst:

2-[4-(4-Fluorphenoxy)-2-fluorphenyl]-1-(1H-1,2,4-triazol-1-yl)-ethan-2-ol,
2-[4-(4-Fluorphenoxy)-2-chlorphenyl]-1-(1H-1,2,4-triazol-1-yl)-ethan-2-ol,
2-[4-(4-Fluorphenoxy)-2-bromphenyl]-1-(1H-1,2,4-triazol-1-yl)-ethan-2-ol,
2-[4-(4-Fluorphenoxy)-2-methylphenyl]-1-(1H-1,2,4-triazol-1-yl)-ethan-2-ol,
2-[4-(4-Chlorphenoxy)-2-fluorphenyl]-1-(1H-1,2,4-triazol-1-yl)-ethan-2-ol,
2-[4-(4-Chlorphenoxy)-2-fluorphenyl]-1-(1H-1,2,4-triazol-1-yl)-ethan-2-ol,
2-[4-(4-Chlorphenoxy)-2-bromphenyl]-1-(1H-1,2,4-triazol-1-yl)-ethan-2-ol,
2-[4-(4-Chlorphenoxy)-2-methylphenyl]-1-(1H-1,2,4-triazol-1-yl)-ethan-2-ol,
2-[4-(4-Bromphenoxy)-2-fluorphenyl]-1-(1H-1,2,4-triazol-1-yl)-ethan-2-ol,
2-[4-(4-Bromphenoxy)-2-chlorphenyl]-1-(1H-1,2,4-triazol-1-yl)-ethan-2-ol,
2-[4-(4-Bromphenoxy)-2-bromphenyl]-1-(1H-1,2,4-triazol-1-yl)-ethan-2-ol,
2-[4-(4-Bromphenoxy)-2-methylphenyl]-1-(1H-1,2,4-triazol-1-yl)-ethan-2-ol,
sowie ihre Salze mit pflanzenphysiologisch verträglichen organischen und anorganischen Säuren.

Die 12 Phenoxyphenyl-1-triazol-carbinole der Formel I sind neu. Verbindungen mit ähnlicher Konstitution und mikrobizid-fungizider Wirkung sind aus EP-A 77 479 bekannt geworden. Es werden in dieser Referenz jedoch nur Diphenylether-Derivate genannt, bei denen der zentrale Phenylring keine weiteren Substituenten enthält. Die erfindungsgemässen Verbindungen, bei denen der zentrale Phenylring durch $R_2$ substituiert ist, zeichnen sich durch sprunghaft verbesserte phytofungizide Wirkung aus und können daher in äusserst geringen Aufwandmengen eingesetzt werden. Aus dieser Referenz konnte der Fachmann keinen Hinweis entnehmen, dass dis erfindungsgemässen Verbindungen der Formel I ausserordentlich wirksame Fungizide darstellen.

Die Herstellung der Phenoxy-triazol-carbinole der Formel I geschieht durch Kondensation eines Phenoxy-acetophenons der Formel II mit 1H-1,2,4-Triazol der Formel III oder einem Metallsalz davon entsprechend der Gleichung

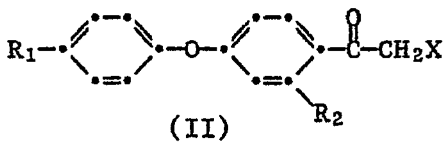 + 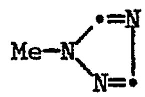

(II) (III)

In diesen Formeln haben $R_1$ und $R_2$ die unter der Formel I gegebene Bedeutung, während Me Wasserstoff oder ein Metallkation, X eine nukleofuge Abgangsgruppe, wie z.B. Halogen, insbesondere Chlor, Brom oder Jod, aber auch eine Benzolsulfonyloxy-, p-Tosyloxy-, Trifluoracetyloxy- oder bevorzugt eine Niederalkylsulfonyloxy- wie Mesyloxygruppe bedeutet. Die entstandenen 2-(Diphenylether)-1-(1H-1,2,4-triazol)-ethan-2-one der Formel IV

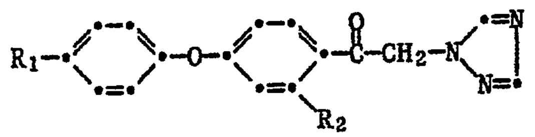 (IV)

worin $R_1$ und $R_2$ die unter der Formel I gegebene Bedeutung haben, werden dann mit Wasserstoff oder einem Wasserstoff abgebenden Mittel zu Verbindungen der Formel I reduziert.

Wird für die Umsetzung ein Acetophenon-halid der Formel II und 1H-1,2,4-Triazol verwendet, so wird die Reaktion vorteilhafterweise n Gegenwart einer Base und einer katalytischen Menge Kaliumjodid in einem Keton als Lösungsmittel vorgenommen.

Das Lösungsmittel sollte wasserfrei sein und kann aus Aceton, Methyläthylketon, Cyclohexanon, bestehen. Neben Ketonen kommen als Lösungsmittel auch Ether wie Diethylether, Dioxan oder Tetrahydrofuran in Frage.

Als Base wird ein tertiäres Amin wie Triäthylamin, Methylpyridin oder aber eine anorganische Base, vorzusweise eine Alkalimetallcarbonat oder -bicarbonat verwendet.

Die Reduktion wird in einem inerten organischen Lösungsmittel in Gegenwart von Wasserstoff oder vorzugsweise einem Wasserstoff abgebenden Mittel wie Natriumborhydrid oder Lithiumaluminiumhydrid durchgeführt.

Bei den Reduktionen oder Hydrierungen der Ketonverbindungen der Formel IV zu Carbinolen der Formel I entstehen stereoisomere R,S-Gemische, deren Zusammensetzung von dem zur Hydrierung verwendeten Katalysator oder vom Reduktionsmittel abhängig ist.

Als Lösungsmittel werden hier die oben erwähnten Ether, oder aber niedere Alkanole wie Methanol, Ethanol, Propanol, Isopropanol verwendet.

Die Reaktionstemperaturen bei diesen Umsetzungen liegen zwischen 0°C und dem Siedepunkt des Reaktionsgemisches.

Die Ausgangsstoffe der Formel II sind bekannt oder können in an sich bekannter Weise durch Kondensation von einem 4-Halogenacetophenon mit einem Phenol respektive einem 4-Hydroxyacetophenon mit Halogenbenzol und anschliessender Halogenierung erhalten werden, entsprechend der Gleichung

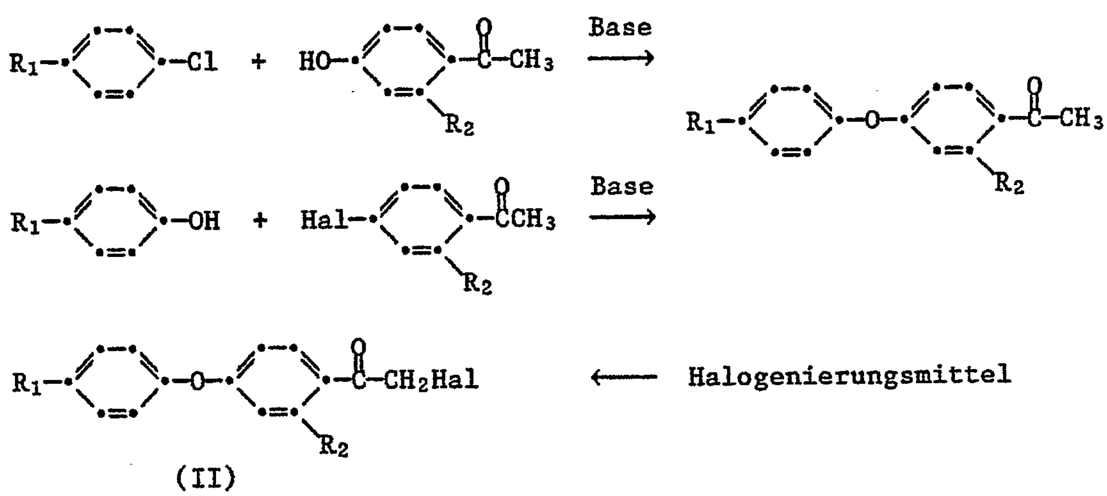


(II)

Die Carbinole der Formel I besitzen ein chirales Zentrum und können in einer R- und S-Form auftreten.

Im allgemeinen entsteht bei der Herstellung dieser Substanzen ein Gemisch beider Enantiomeren. Dieses lässt sich auf übliche Weise, z.B. durch fraktionierte Kristallisation von Salzen optisch aktiver,

starker Säuren, in die reinen optischen Antipoden aufspalten. Beide Enantiomere können unterschiedliche biologische Aktivitäten aufweisen.

Die vorliegende Erfindung betrifft alle reinen Enantiomeren sowie deren Gemische untereinander.

Es wurde überraschend gefunden, dass die Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikrooganismen verschont bleiben.

Die Wirkstoffe der Formel I sind gegen die den folgenden Klassen angehörenden phytopathogenen Pilze wirksam: Fungi imperfecti (z.B. Botrytis, Helminthosporium, Fusarium septoria, Cercospora und Alternaria); Basidiomyceten (z.B. die Gattungen Hemileia, Rhizoctonia, Puccinia); ferner wirken sie gegen die Klasse der Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingessetzt werden.

Die erfindungsgemässen Wirkstoffe zeichnen sich durch besonders gute Pflanzenverträglichkeit aus.

Die Erfindung betrifft auch die Verwendung der Verbindungen der Formel I zur Bekämpfung phytopathogener Mikroorganismen, insbesondere pflanzenschädigender Pilze bzw. die präventive Verhütung eines Befalls von Pflanzen.

Darüber hinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung z.B. folgende Pflanzenarten: Getreide: (Weizen, Gerste, Roggen, Hafer, Reis, Sorghum und Verwandte); Rüben: (Zucher- und Futterrüben); Kern-, Stein- und Beerenobst: (Aepfel, Birnen, Pflaumen, Pfirsiche, Mandeln, Kirschen, Erdbeeren, Himbeeren, Preiselbeeren und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusrüchte: (Orangen, Zitronen, Grapefruit, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Ananas, Tee, Weineben, Hopfen, Bananen- und Naturkautschukegewächse sowie Zierpflanzen (Blumen, Sträucher, Laubbäume und Nadelbäume wie Koniferen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelment-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft,-Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entspechende Phosphate wie z.B. Salze des Phosphorsäureesters eines p-Nonylphenyl-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlensoffatome im (aliphatischen) Kohlenwasserstoffrest und 6 bis 18 Kohlenstoffatome im Alkylrest der Alkylphenole enthalten können.

Weiter geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Aklylkette. Die genannten Verbindungen enthalten üblicherweise pro Propylenglykol- Einheit 1 bis 5 Ethylenglykoleinheiten.

Als Beispiele nichtionischer Tenside seien Nonylphenylpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welches als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbrimid.

Die agrochemischen Zubereitungen enthalten in der Regel 0,1 bis 99 Gew.-% insbesondere 0,1 bis 95

Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Das nachfolgende Beispiel beschreibt die Herstellung eines Wirkstoffes der Formel I. Temperaturen sind in diesem Beispiel in Celsiusgraden angegeben. Drücke in Millibar (mbar).

1. Herstellungsbeispiele

1.1 Herstellung von 2-[4-(4-Bromphenoxy)-2-chlorphenyl]-1-(1H-1,2,4-triazol-1-yl)ethan-2-ol

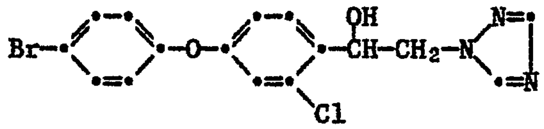

Zu einer Lösung von 5.8 g (14.7 mMol) 2-[4-(4-Bromphenoxy)-2-chlorphenyl]-1-(1H-1,2,4-triazol-1-yl)ethan-2-on in 40 ml Methanol gibt man unter Rühren und Durchleiten von Stickstoff bei Raumtemperatur portionenweise 0.56 g (14.7 mMol) Natriumborhydrid innerhalb 15 Minuten. Dabei erwärmt sich das Reaktionsgemisch von 220° auf 38°. Nachdem alles zugegeben ist, lässt man während 4 Stunden bei Raumtemperatur weiterrühren, gibt dann 2,2 g konz. Salzsäure und etwas später 22 ml 10 %ige Natriumbicarbonatlösung zu. Das Titelprodukt fällt dabei kristallin aus. Es wird abfiltriert, mit Wasser gewaschen und getrocknet. Man erhält so 5.5 g 2-[4-(4-Bromphenoxy)-2-chlorphenyl]-1-(1H-1.2.4-triazol-1-yl)ethan-2-ol als beiges kristallines Pulver. Schmelzpunkt 115 - 118°C.

Das als Ausgangsprodukt benötigte 2-[4-(4-Bromphenoxy)-2-chlorphenyl]-1-(1H-1,2,4-triazol-1-yl)-ethan-2-on wird wie folgt hergestellt:

Ein Gemisch von 8.7 g (21.5 mMol) 2-[4-(4-Bromphenoxy)-2-chlorphenyl]-1-bromethan-2-on, 1.63 g (23.7 mMol) 1,2,4-Triazol, 3.26 g (23.7 mMol) Kaliumcarbonat und eine katalytisch wirkende Menge von 0.3 g Kaliumjodid in 80 ml absolutem Butan-2-on wird während 24 Stunden bei Raumtemperatur gerührt. Dann gibt man nochmals 0.15 g (2.1 mMol) 1,2,4-Triazol zu und erwärmt während 6 Stunden unter Rühren auf 45°. Nach dem Abkühlen auf Raumtemperatur wird das Reaktionsgemisch filtriert, das Filtrat mit Aktivkohle behandelt, wieder filtriert und am Rotationsverdampfer eingeengt. Der Rückstand wird aus Ethanol umkristallisiert. Es verbleibt ein beige-weisses kristallines Pulver von 2-[4-(4-Bromphenoxy)-2-chlor-phenyl]-(1H-1,2,4-triazol)ethan-2-on, welches bei 171 - 173° schmilzt.

In gleicher Weise werden die in der Tabelle 1 aufgeführten Verbindungen hergestellt.

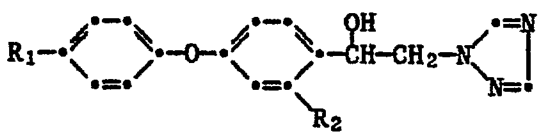

Tabelle 1

| No. | $R_1$ | $R_2$ | physikalische Daten |
|---|---|---|---|
| 1. | F | F | Smp. 117 – 119° |
| 2. | F | Cl | Smp. 110 – 111° |
| 3. | F | Br | Smp. 115 – 117° |
| 4. | F | $CH_3$ | Smp. 102 – 103° |
| 5. | Cl | F | |
| 6. | Cl | Cl | Smp. 94 – 96° |
| 7. | Cl | Br | Smp. 108 – 110° |
| 8. | Cl | $CH_3$ | Smp. 107 – 108° |
| 9. | Br | F | |
| 10. | Br | Cl | Smp. 115 – 118° |
| 11. | Br | Br | Smp. 115 – 117° |
| 12. | Br | $CH_3$ | Smp. 119 – 121° |

2. Formulierungsbeispiele für feste Wirkstoffe der Formel I (% = Gewichtsprozent)

| 2.1 Spritzpulver | a) | b) | c) |
|---|---|---|---|
| Wirkstoffe aus Tabelle 1 | 25 % | 50% | 75% |
| Na-Ligninsulfonat | 5% | 5% | |
| Na-Laurylsulfat | 3% | – | 5% |
| Na-Diisobutylnaphthalinsulfonat | – | 6% | 10% |
| Octylphenolpolyethylenglykolether (7–8 Mol Ethylenoxid) | – | 2% | – |
| Hochdisperse Kieselsäure | 5% | 10% | 10% |
| Kaolin | 62% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.2 Emulsions-Konzentrat Wirkstoff aus Tabelle 110 %
Octylphenolpolyethylenglykolether (4-5 Mol Ethylenoxid)3 %
Ca-Dodecylbenzolsulfonat3 %
Ricinusölpolyglykoleter (35 Mol Ethylenoxid)4 %

| 2.3 Stäubemittel | a) | b) |
|---|---|---|
| Wirkstoffe aus Tabelle 1 | 5% | 8% |
| Talkum | 95% | – |
| Koalin | – | 92% |

Man erhält anwendungsfertige Stäubelmittel, indem der Wirkstoff mit den Träger vermischt und auf einer geeigneten Mühle vermahlen wird.

2.4 Extruder Granulat Wirkstoff ans Tabelle 110 %
Na-Ligninsulfonat2 %
Carboxymethylcellulose1 %
Kaolin87 %

Der Wirkstoff wird mit den Zusatzstoffen vermischt, vermahlen und mit Wasser angefeuchtet. Dieses Gemisch wird extrudiert und anschliessend im Luftstrom getrocknet.

2.5 Umhüllungs-Granulat Wirkstoff aus Tabelle 13 %
Polyethylenglykol (MG 200)3 %
Kaolin94%
(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6 Suspensions-Konzentrat Wirkstoff aus Tabelle 140 %
Ethylenglykol10 %
Nonylphenolpolyethylenglykolether (15 Mol Ethylenoxid)6 %
N-Ligninsulfonat10 %
Carboxymethylcellulose1 %
37%ige wässrige Formaldehyd-Lösung0,2 %
Silikonöl in Form einer 75%igen wässrigen Emulsion0,8 %
Wasser32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele:

Beispiel 3.1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,006 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,002 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Die Verbindungen der Tabelle 1 verhindern den Puccinia-Befall fast vollständig. (Befall = 0 - 10 %). Unbehandelte aber infizierte Kontrollpflanzen weisen dagegen einen Puccinia-Befall von 100% auf.

Beispiel 3.2: Wirkung gegen Cercospora arachidicola auf Erdnusspflanzen

Residual-protektive Wirkung

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden währen 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigen Erdnusspflanzen, die mit Wirkstoffen aus der Tabelle behandelt wurden, einen stark reduzierten Cercospora-Befall. So verhindern z.B. die Verbindungen der Tabelle 1 das Auftreten von Flecken fast vollständig (Befall = 0 - 10 %).

Beispiel 3.3: Wirkung gegen Erysiphe graminis auf Gerste

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen aus der Tabelle 1 zeigen gute Wirksamkeit gegen Erysiphe-Pilze. Unbehandelte jedoch infizierte Kontrollpflanzen weisen dagegen einen Erysiphe-Befall von 100 % auf.

Beispiel 3.4: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,06 % Aktivsubstanz) besprüht. Nach 24 Stunden werden die behandelten Pflanzen mit einer Konidiensuspension des Pilzes infiziert. Die Pflanzen werden dann während 5 Tage bei 90-100 % relativer Luftfeuchtigkeit inkubiert und während 10 weiterer Tage in einem Gewächshaus bei 20-24°C aufgestellt. Der Schorfbefall wird 15 Tage nach der Infektion beurteilt. Verbindungen aus der Tabelle zeigen gute Wirksamkeit gegen Venturia. Unbehandelte jedoch infizierte Triebe weisen dagegen einen 100%igen Venturia-Befall auf.

Beispiel 3.5: Wirkung gegen Botrytis cinerea auf Bohnen

Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tage bei 95-100 % relativer Luftfeuchtigkeit und 21°C erfolgt die Beurteilung des Pilzbefalls. Verbindungen aus der Tabelle hemmen die Pilzinfektion sehr stark. Unbehandelte jedoch infizierte Kontrollpflanzen weisen dagegen einen Botrytis-Befall von 100 % auf.

Beispiel 3.6: Wirkung gegen Tilletia caries an Weizen

Künstlich mit Brandsporen von Tilletia caries infizierte Wintergerste der Sorte Probus (3 g trockenes Sporenmaterial auf 1 kg Saatgut) wird auf einer Mischrolle mit dem zu prüfenden Fungizid gebeizt, wobei eine Konzentration von 60 ppm AS (bezogen auf das Gewicht des Saatgutes) zur Anwendung gelangt.

Der infizierte und behandelte Weizen wird im Oktober im Freiland mittels einer Sämaschine auf Parzellen von 2 m Länge und 3 Saatreihen mit dreifacher Wiederholung ausgesät.

Zur Ermittlung der Wirkstoffaktivität werden zum Zeitpunkt der Aehrenreife der prozentuale Anteil Tilletia-befallener Aehren ausgezählt.

Die Verbindungen aus der Tabelle 1 zeigen gute Wirksamkeit gegen Tilletia und hemmen den Pilzbefall bis auf 0 - 5 %, unbehandelte jedoch infizierte Kontrollpflanzen weisen dagegen einen Tilletia-Befall von 100 % auf.

Beispiel 3.7: Wirkung gegen Helminthosporium gramineum an Gerste

Auf natürliche Weise mit Helminthosporium gramineum infizierte Wintergerste der Sorte "C1" wird auf einer Mischrolle mit dem zu prüfenden Fungizid gebeizt, wobei eine Konzentration von 60 ppm AS (bezogen auf das Gewicht des Saatgutes) zur Anwendung gelangt.

Die infizierte und behandelte Gerste wird im Oktober im Freiland mittels einer Sämaschine auf Parzellen von 2 m Länge und 3 Saatreihen mit dreifacher Wiederholung ausgesät.

Biz zur Befallsauswertung wird die Versuchspflanzung unter normalen Feldbedingungen kultiviert.

Zur Ermittlung der Wirkstoffaktivität werden zum Zeitpunkt des Aehrenschiebens der prozentuale Anteil Helminthosporium-befallener Halme ausgezählt.

Die Verbindungen der Tabelle 1 zeigen gute Wirksamkeit gegen Helminthosporium. Sie hemmen den Pilzbefall auf 0 - 5 %, während unbehandelte infizierte Kontrollpflanzen einen Helminthosporium-Befall von 100 % aufweisen.

Beispiel 3.8:

Gemäss den in den vorausgehend beschriebenen biologischen Beispielen durchgeführten Tests wurden folgende strukturell nächststehende Verbindungen aus dem Stand der Technik im Vergleich mit Verbindungen der vorliegenden Erfindung geprüft.

Verbindungen aus dem Stand der Technik der Formel

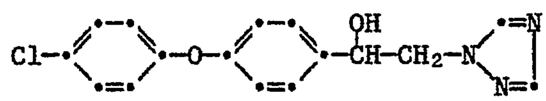

(A)

bekannt aus EP-A 77 479 No. 67

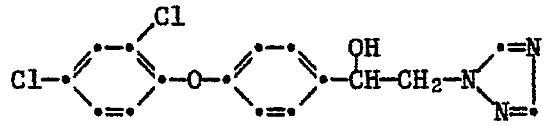

(B)

bekannt aus EP-A 77 479 No. 66.

Biologische Tests (Residuale Wirkung)

1. Bewertungsskala

| Note | %-Aktivität | Pilzbefall |
|---|---|---|
| 1 | ≥ 95 | 0–5% |
| 3 | 80–95 | 5–20% |
| 6 | 50–80 | 20–50% |
| 9 | ≤ 50 | ≥ 50% |

Eine Verbindung gilt als unwirksam, wenn der Pilzbefall an der Pflanze 50 % oder mehr beträgt.

2. Testergebnisse

| Verb | Puccinia 200/20 ppm | | Cercospora 200/20 ppm | | Erysiphe 200/20 ppm | | Venturia 200/60 ppm | |
|---|---|---|---|---|---|---|---|---|
| A | 9 | 9 | 9 | 9 | 3 | 6 | 9 | 9 |
| B | 5 | 9 | 9 | 9 | 1 | 5 | 9 | 9 |
| 1 | 2 | 2 | 1 | 9 | 1 | 1 | 5 | 9 |
| 2 | 3 | 6 | 1 | 3 | 1 | 3 | 9 | 9 |
| 4 | 3 | 3 | 5 | 5 | 3 | 5 | 1 | 5 |
| 6 | 1 | 1 | 1 | 2 | 1 | 1 | 1 | 1 |
| 7 | 2 | 3 | 1 | 1 | 1 | 2 | 1 | 1 |
| 8 | 3 | 3 | 1 | 5 | 3 | 5 | 3 | 3 |
| 10 | 2 | 2 | 1 | 3 | 1 | 1 | 1 | 1 |

**Patentansprüche für die Vertragsstaaten: AT, BE, CH, DE, FR, GB, GR, IT, LI, LU, NL, SE**

1. 1-Phenoxyphenyl-1-triazolylmethyl-carbinole der Formel I

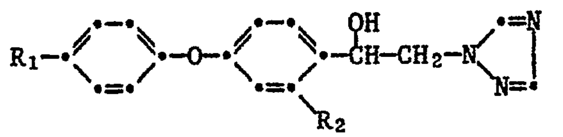

(I)

worin
$R_1$ Fluor, Chlor oder Brom
$R_2$ Fluor, Chlor, Brom oder Methyl bedeuten
sowie die Säureadditionssalze mit pflanzenphysiologisch unbedenklichen organischen und anorganischen Säuren.

2. Eine Verbindung gemäss Anspruch 1 ausgewählt aus der Gruppe: 2-[4-(4-Fluorphenoxy)-2-fluorphenyl]-1-(1H-1,2,4-triazol-1-yl)-ethan-2-ol;
2-[4-(4-Fluorphenoxy)-2-chlorphenyl]-1-(1H-1,2,4-triazol-1-yl)-ethan-2-ol;
2-[4-(4-Fluorphenoxy)-2-methylphenyl]-1-(1H-1,2,4-triazol-1-yl)-ethan-2-ol;
2-[4-(4-Chlorphenoxy)-2-chlorphenyl]-1-(1H-1,2,4-triazol-1-yl)-ethan-2-ol;
2-[4-(4-Chlorphenoxy)-2-bromphenyl]-1-(1H-1,2,4-triazol-1-yl)-ethan-2-ol;
2-[4-(4-Chlorphenoxy)-2-methylphenyl]-1-(1H-1,2,4-triazol-1-yl)-ethan-2-ol;
2-[4-(4-Bromphenoxy)-2-chlorphenyl]-1-(1H-1,2,4-triazol-1-yl)-ethan-2-ol;
2-[4-(4-Bromphenoxy)-2-bromphenyl]-1-(1H-1,2,4-triazol-1-yl)-ethan-2-ol;

3. Verfahren zur Herstellung der 1-Phenoxyphenyl-1-triazolylmethylcarbinole der Formel I

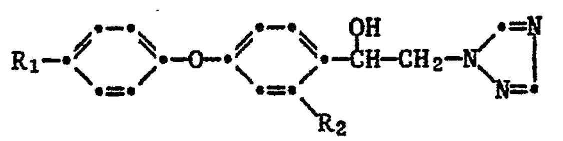

(I)

worin $R_1$ Fluor, Chlor oder Brom und
$R_2$ Fluor, Chlor, Brom oder Methyl
bedeuten und deren Säureadditionssalzen, dadurch gekennzeichnet, dass man 1H-1,2,4-Triazol oder ein Metallsalz davon mit einem 4-Phenoxy-acetophenon der Formel II kondensiert

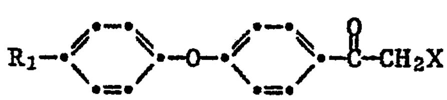

(II)

worin $R_1$ und $R_2$ die oben gegebene Bedeutung haben und X eine nukleofuge Abgangsgruppe bedeutet, und das entstandene 2-(Diphenylether)-1-(1H-1,2,4-triazol)ethan-2-on der Formel III

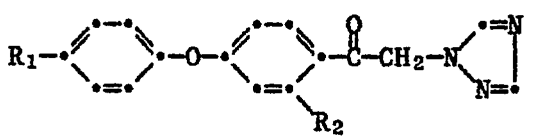

(IV)

worin $R_1$ und $R_2$ die oben gegebene Bedeutung haben, mit Wasserstoff oder einem Wasserstoff abgebenden Mittel reduziert.

4. Mittel zur Bekämpfung oder Verhütung eines Befalles durch Mikroorganismen, dadurch gekennzeichnet, dass es neben inerten Formulierungshilfsstoffen als aktive Komponente eine wirksame Menge eines 1-Phenoxyphenyl-1-triazolylmethyl-carbinols der Formel I

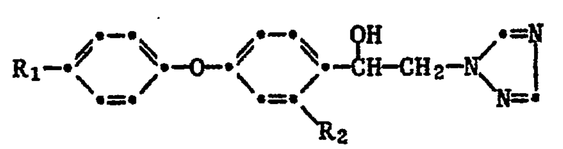

(I)

worin $R_1$ Fluor, Chlor oder Brom und
$R_2$ Fluor, Chlor, Brom oder Methyl darstellen oder
eines pflanzenphsiologisch verträglichen Säureadditionsalzes einer solchen Verbindung enthält.

5. Mittel gemäss Anspruch 4, dadurch gekennzeichnet, dass es als alktive Komponente mindestens eine Verbindung der Formel I gemäss Anspruch 2 enthält.

6. Mittel nach einem der Ansprüche 4 und 5, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

7. Mittel nach Anspruch 6, dadurch gekennzeichnet, dass es 0,1 bis 95 % eines Wirkstoffs der Formel I, 99,8 bis 5 % eines festen oder flüssigen Zusatzstoffes und 0,1 bis 25 % eines Tensides enthält.

8. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss Anspruch 1 oder 2 definierte Verbindung der Formel I auf die Pflanze oder deren Standort appliziert.

9. Verwendung von Verbindungen der Formel I gemäss Anspruch 1 oder sie enthaltender Mittel zur Bekämpfung und/oder zur präventiven Verhütung eines Befalls von Mikroorganismen.

10. Verfahren gemäss Anspruch 8, dadurch gekennzeichnet, dass es sich bei den Mikroorganismen um phytopathogene Pilze handelt.

**Patentansprüche für den Vertragsstaat: ES**

1. Verfahren zur Herstellung der 1-Phenoxyphenyl-1-triazolyl-methyl-carbinole der Formel I

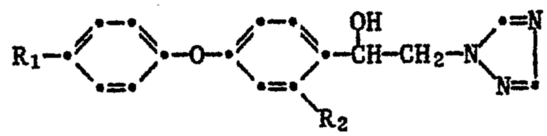

(I)

worin $R_1$ Fluor, Chlor oder Brom und
$R_2$ Fluor, Chlor, Brom oder Methyl
bedeuten, und deren Säureadditionssalze, dadurch gekennzeichnet, daß man 1H-1,2,4-Triazol oder ein Metallsalz davon mit einem 4-Phenoxy-acetophenon der Formel II kondensiert

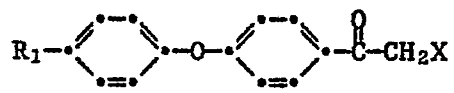

(II)

worin $R_1$ und $R_2$ die oben angegebene Bedeutung haben und X eine nukleofuge Abgangsgruppe bedeutet, und das entstandene 2-(Diphenylether)-1-(1H-1,2,4-triazol)ethan-2-on der Formel (IV)

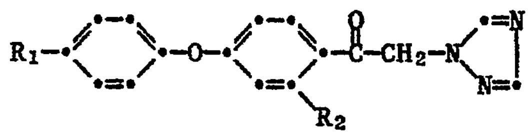

(IV)

worin $R_1$ und $R_2$ die oben angegebene Bedeutung haben, mit Wasserstoff oder einem Wasserstoff abgebenden Mittel reduziert.

2. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, daß man eine gemäß Anspruch 1 definierte Verbindung der Formel I auf die Pflanze oder deren Standort appliziert.

3. Verfahren gemäß Anspruch 2, dadurch gekennzeichnet, daß es sich bei den Mikroorganismen um phytopathogene Pilze handelt.

**Claims for the Contracting States: DE, GB, FR, IT, NL, SE, LI, CH, BE, AT, LU, GR**

1. 1-phenoxyphenyl-1-triazolylmethyl-carbinols of formula I

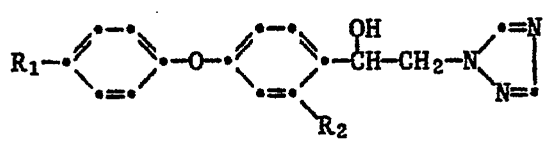

(I)

in which
$R_1$ is fluorine, chlorine or bromine,
$R_2$ is fluorine, chlorine, bromine or methyl, and the acid addition salts with phytophysiologically tolerable organic and inorganic acids.

2. A compound according to claim 1 selected from the group of
2-[4-(4-fluorophenoxy)-2-fluorophenyl]-1-(1H-1,2,4-triazol-1-yl)-ethan-2-ol;
2-[4-(4-fluorophenoxy)-2-chlorophenyl]-1-(1H-1,2,4-triazol-1-yl)-ethan-2-ol;
2-[4-(4-fluorophenoxy)-2-methylphenyl]-1-(1H-1,2,4-triazol-1-yl)-ethan-2-ol;

2-[4-(4-chlorophenoxy)-2-bromophenyl]-1-(1H-1,2,4-triazol-1-yl)-ethan-2-ol;
2-[4-(4-chlorophenoxy)-2-methylphenyl]-1-(1H-1,2,4-triazol-1-yl)-ethan-2-ol;
2-[4-(4-bromophenoxy)-2-chlorophenyl]-1-(1H-1,2,4-triazol-1-yl)-ethan-2-ol;
2-[4-(4-bromophenoxy)-2-bromophenyl]-1-(1H-1,2,4-triazol-1-yl)-ethan-2-ol.

3. A process for the preparation of the 1-phenoxyphenyl-1-triazolylmethyl-carbinols of formula I

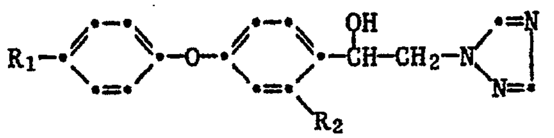
(I)

in which $R_1$ is fluorine, chlorine or bromine and $R_2$ is fluorine, chlorine, bromine or methyl and acid addition salts thereof, characterised in that 1H-1,2,4-triazole or a methal salt thereof is condensed with a 4-phenoxy-acetophenone of formula II

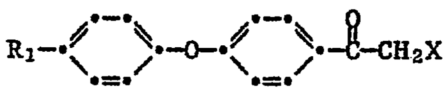
(II)

in which $R_1$ and $R_2$ have the meanings given above and X is a nucleofugal leaving group, and the resulting 2-(diphenyl ether)-1-(1H-1,2,4-triazol)-ethan-2-one of formula IV

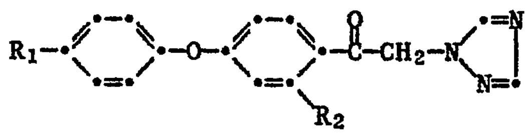
(IV)

in which $R_1$ and $R_2$ have the meanings given above is rduced with hydrogen or a hydrogen donor.

4. A composition for controlling or preventing attacks by microorganisms, characterised in that it contains, as active component, an effective amount of a 1-phenoxy-phenyl-1-triazolylmethyl-carbinol of formula I

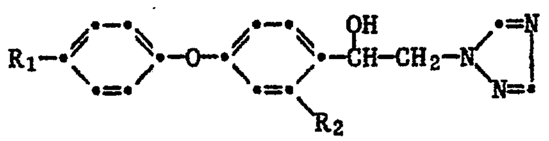
(I)

in which $R_1$ is fluorine, chlorine or bromine and $R_2$ is fluorine, chlorine, bromine or methyl, or a phytophysiologically tolerable acid addition salt of such a compound, together with inert formulation adjuvants.

5. A composition according to claim 4, characterised in that it contains, as active component, at least one compound of formula I according to claim 2.

6. A composition according to one of claims 4 and 5, characterised in that it contains 0.1 to 99% of a compound of formula I, 99.9 to 1% of a solid or liquid adjuvant and 0 to 25% of a surfactant.

7. A composition according to claim 6, characterised in that it contains 0.1 to 95% of a compound of formula I, 99.8 to 5% of a solid or liquid adjuvant and 0.1 to 25% of a surfactant.

8. A method of controlling or preventing attacks on cultivated plants by phytopathogenic microorganisms and/or for regulating plant growth, characterised in that a compound of formula I defined according to claim 1 or 2 is applied to the plant or its locus.

9. The use of compounds of formula I according to claim 1 or of compositions containing them for controlling and/or preventing attachs by microorganisms.

10. A method according to claim 8, characterised in that the microorganisms are phytopathogenic fungi.

**Claims for the Contracting State: ES**

1. A process for the preparation of the 1-phenoxyphenyl-1-triazolylmethyl-carbinols of formula I

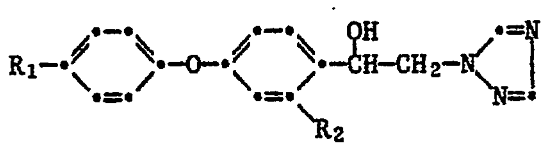
(I)

in which $R_1$ is fluorine, chlorine or bromine and $R_2$ is fluorine, chlorine or bromine or methyl and acid addition salts thereof, characterised in that 1H-1,2,4-triazole or a metal salt thereof is condensed with a 4-phenoxy-acetophenone of formula II

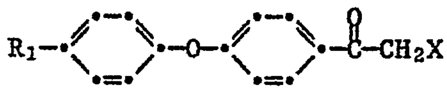 (II)

in which $R_1$ and $R_2$ have the meanings given above and X is a nucleofugal leaving group, and the resulting 2-(diphenyl ether)-1-(1H-1,2,4-triazol)-ethan-2-one of formula IV

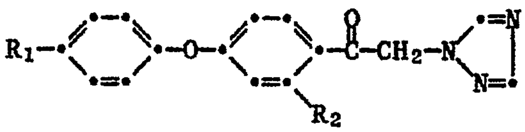 (IV)

in which $R_1$ and $R_2$ have the meanings given above is reduced with hydrogen or a hydrogen donor.

2. A method of controlling or preventing attachs on cultivated plants by phytopathogenic microorganisms and/or for regulating plant growth, characterised in that a compound of formula I defined according to claim 1 is applied to the plant or its locus.

3. A method according to claim 2, characterised in that the microorganisms are phytophathogenic fungi.

**Revendications pour les Etats Contractants: DE, GB, FR, IT, NL, SE, LI, CH, BE, AT, LU, GR**

1. 1-phénoxyphényl-1-triazolylméthyl-carbonols de formule I

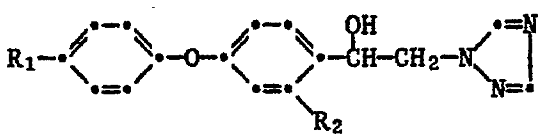 (I)

où
$R_1$ représente un fluor, chlore ou brome,
$R_2$ représente un fluor, chlore, brome ou méthyle, ainsi que les sels d'addition d'acides avec des acides organiques ou inorganiques compatibles avec la physiologie des plantes.

2. Composé selon la revendication 1, choisi dans le groupe:
2-[4-(4-fluorophénoxy)-2-fluorophényl]-1-(1H-1,2,4-triazol-1-yl)-éthan-2-ol;
2-[4-(4-fluorophénoxy)-2-chlorophényl]-1-(1H,1,2,4-triazol-1-yl)-éthan-2-ol;
2-[4-(4-fluorophénoxy)-2-méthylphényl]-1-(1H-1,2-4-triazol-1-yl)-éthan-2-ol;
2-[4-(4-chlorophénoxy)-2-chlorophényl]-1-(1H-1,2,4-triazol-1-yl)-éthan-2-ol;
2-[4-(4-chlorophénoxy)-2-bromophényl]-1-(1H-1,2,4-triazol-1-yl)-éthan-2-ol;
2-[4-(4-chlorophénoxy)-2-méthylphényl]-l-(1H-1,2,4-triazol-1-yl)-éthan-2-ol;
2-[4-(4-bromophénoxy)-2-chlorophényl]-1-(1H-1,2,4-triazol-1-yl)-éthan-2-ol;
2-[4-(4-bromophénoxy)-2-bromophényl]-1-(1H-1,2,4-triazol-1-yl)-éthan-2-ol.

3. Préparation des 1-phénoxyphényl-1-triazolylméthyl-carbinols de formule I

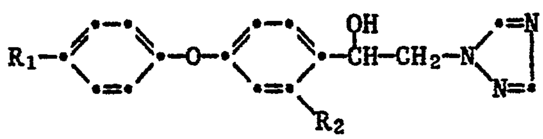 (I)

où
$R_1$ représente un fluor, chlore ou brome, et
$R_2$ représente un fluor, chlore, brome ou méthyle et de leurs sels d'addition d'acides, caractérisée en ce qu'on condense un 1H-1,2,4-triazole ou un de ses sels métalliques avec une 4-phénoxy-acétophénone de formule II

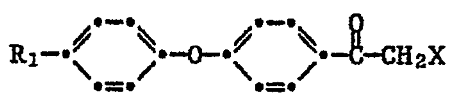 (II)

où $R_1$ et $R_2$ ont la signification donnée ci-dessus et X représente un groupe sortant nucléofuge, et la 2-(diphényléther)-1-(1H-1,2,4-triazol)éthan-2-one de formule IV

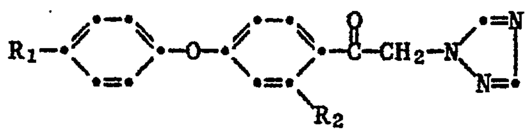

(IV)

où $R_1$ et $R_2$ ont la signification donnée ci-dessus, avec de l'hydrogène ou un agent donnant de l'hydrogène.

4. Agent pour combattre ou prévenir une attaque par les microorganismes, caractérisé en ce qu'il contient outre des additifs de formulation inerte, comme composant actif, une quantité efficace d'un 1-phénoxyphényl-1-triazolylméthyl-carbinol de formule I

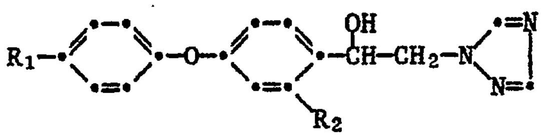

(I)

où
$R_1$ représente un fluor, chlore ou brome et
$R_2$ représente un fluor, chlore, brome ou méthyle ou un sel d'addition d'acides acceptable pour la physiologie des plantes d'un tel composé.

5. Agent selon la revendication 4, caractérisé en ce qu'il contient comme composant actif au moins un composé de formule I selon la revendication 2.

6. Agent selon l'une des revendications 4 ou 5, caractérisé en ce qu'il contient de 0,1 à 99% de matière active de formule I, de 99,9 à 1% d'un agent tensio-actif.

7. Agent selon la revendication 6, caractérisé en ce qu'il contient de 0,1 à 95% de matière active de formule I, 99,8 à 5% d'un additif solide ou liquide et 0,1 à 25% d'un agent tensio-actif.

8. Procédé pour combattre ou prévenir une attaque des plantes cultivées par des microorganismes phytophathogènes et/ou pour réguler la croissance végétale, caractérisé en ce qu'on applique un composé de formule I défini selon la revendication 1 ou 2 sur la plante ou l'endroit où elle se trouve.

9. Application de composés de formule I selon la revendication 1 ou des agents qui les contiennent pour combattre et/ou prévenir une attaque de microorganismes.

10. Procédé selon la revendication 8, caractérisé en ce qu'il s'agit pour les microorganismes de champignons phytophathogènes.

**Revendications pour l'Etat Contractant: ES**

1. Préparation des 1-phénoxyphényl-1-triazolylméthyl-carbinols de formule I

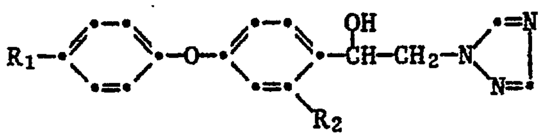

(I)

où
$R_1$ représente un fluor, chlore ou brome, et
$R_2$ représente un fluor, chlore, brome ou méthyle
et de leurs sels d'addition d'acides, caractérisée en ce qu'on condense un 1H-1,2,4-triazole ou un de ses sels métalliques avec une 4-phénoxy-acétophénone de formule II

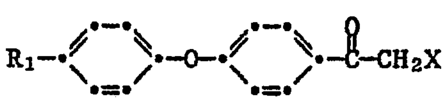

(II)

où $R_1$ et $R_2$ ont la signification donnée ci-dessus et X représente un groupe sortant nucléofuge, et la 2-(diphényléther)-1-(1H-1,2,4-triazol)éthan-2-one de formule IV

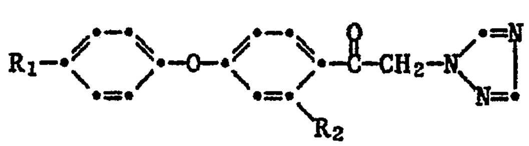

(IV)

où $R_1$ et $R_2$ ont la signification donnée ci-dessus, avec de l'hydrogène ou un agent donnant de l'hydrogène.

2. Procédé pour combattre ou prévenir une attaque des plantes cultivées par des microorganismes phytopathogènes et/ou pour réguler la croissance végétale, caractérisé en ce qu'on applique un composé de formule I défini selon la revendication 1 sur la plante ou l'endroit où elle se trouve.

3. Procédé selon la revendication 2, caractérisé en ce qu'il s'agit pour les microorganismes de champignons phytophathogènes.